# FASCICULE DE BREVET EUROPEEN

(11) **EP 3 648 672 B1**
(45) Date de publication et mention de la délivrance du brevet: **28.02.2024**
(21) Numéro de dépôt: 18734255.5
(22) Date de dépôt: 04.07.2018
(51) Int. Cl.: A61B 6/04, A61B 5/00, A61B 90/14, A61G 13/12

(54) **DISPOSITIF MÉDICAL POUR CONTRAINDRE UN SEGMENT ARTICULAIRE HUMAIN, SYSTÈME CORRESPONDANT, PROCÉDÉ ET UTILISATIONS ASSOCIÉS**
MEDIZINISCHE VORRICHTUNG ZUR DRUCKAUSÜBUNG AUF EIN MENSCHLICHES GELENKSEGMENT, ENTSPRECHENDES SYSTEM, ZUGEHÖRIGES VERFAHREN UND VERWENDUNGEN
MEDICAL DEVICE FOR APPLYING PRESSURE TO A HUMAN JOINT SEGMENT, CORRESPONDING SYSTEM, ASSOCIATED METHOD AND USES

(30) Priorité: 04.07.2017 FR 1756289
(43) Date de publication de la demande: 13.05.2020
(73) Titulaire: Université de Poitiers, 86000 Poitiers (FR); Centre National de la Recherche Scientifique, 75016 Paris Cedex 16 (FR); Centre Hospitalier Universitaire de Poitiers, 86000 Poitiers (FR); Université de Tours, 37020 Tours (FR); Centre Hospitalier Régional Universitaire de Tours, 37000 Tours (FR)
(72) Inventeur: GERMANEAU, Arnaud, 86550 Mignaloux Beauvoir (FR); VENDEUVRE, Tanguy, 86000 Poitiers (FR); BONNARD, Christian, 37700 Saint Pierre Des Corps (FR); BATISSE, François, 63110 Beaumont (FR)
(74) Mandataire: Vidon Brevets & Stratégie
(86) Numéro de dépôt international: PCT/EP2018/068065
(87) Numéro de publication internationale: WO 2019/008020

(56) Documents cités:
- WO-A1-2005/034827
- WO-A2-98/35643
- US-A1- 2011 170 671

## Description

### 1. Domaine de l'invention

L'invention concerne un dispositif médical pour contraindre un segment articulaire humain. L'invention concerne également un système correspondant, un procédé et des utilisations associées. L'invention est exposée dans le jeu de revendications ci-joint.

### 2. Art antérieur

Une articulation est une zone de jonction entre deux extrémités osseuses. Les articulations sont plus ou moins mobiles selon leur constitution, leur forme, et la nature des éléments environnants.

Des déformations constitutionnelles ou post-traumatiques des articulations sont souvent source de gêne, de douleurs et/ou font présenter un risque accru de blessure. Ces déformations peuvent notamment être suivies par radiographie aux rayons X. En particulier, l'étude de la possibilité pour une articulation d'effectuer des mouvements soit d'une amplitude anormale, soit n'existant pas à l'état naturel - également appelé laxité articulaire - est cruciale pour mieux comprendre la mécanique articulaire d'un patient. Elle est une étape très importante précédant une éventuelle action de correction, notamment une correction par voie chirurgicale.

Parmi les anomalies des articulations particulièrement fréquentes se trouvent les déviations sinueuses de la colonne vertébrale, encore appelées scolioses lorsque les déformations ont lieu dans les trois plans de l'espace. Les scolioses idiopathiques de l'adolescent (SIA) peuvent généralement être corrigées par voie chirurgicale. Le but d'une opération chirurgicale est alors d'obtenir un rachis équilibré, stable et centré au-dessus du bassin en réalisant le minimum de fusion vertébrale tout en conservant le maximum de mobilité adjacente.

L'évaluation de la réductibilité des courbures des SIA est une obligation dans le bilan pré-opératoire pour planifier les niveaux d'instrumentation et d'arthrodèse. Il existe de nombreuses méthodes radiographiques pré-opératoires pour évaluer la réductibilité des courbures, dont les clichés radiographiques debout ou couché en flexion latérale active (communément dénommé « *supine side bending* »), les radiographies en flexion sur billot (communément dénommé « *fulcrum bending radiographs* » et « *Changai fulcrum bending radiographs* »), les radiographies en traction avec ou sans anesthésie générale (communément dénommé « *traction radiographs* »), les radiographies avec manoeuvre de réduction par poussée (communément dénommé « *push prone radiographs* ») pouvant être associées aux tractions, ainsi que les radiographies en suspension.

Les clichés radiographiques couchés en flexion latérale active sont actuellement considérés comme la méthode radiographique pré-opératoire de référence. Certains chirurgiens restent cependant réticents à leur utilisation en raison de la nécessité d'une participation active du patient, rendant la technique peu précise, peu fiable et peu reproductible. Cette méthode a également démontré son pouvoir prédictif concernant les résultats avec une instrumentation d'Harrington, mais aucune étude n'a prouvé leur efficacité avec les instrumentations plus modernes.

Un système comprenant trois ou quatre unités d'appui disposées sur les bords latéraux d'une table radiographique horizontale, et permettant d'étudier la flexibilité d'une colonne vertébrale d'un sujet et/ou de planifier le type de correction à apporter par cintrage au niveau de trois ou quatre points de contact, a été décrit dans US 2011/0170671. Chaque unité d'appui semble comprendre un bras extensible dont la partie distale est munie d'un coussinet destiné à être en contact avec une partie du corps humain. Le bras extensible serait muni de capteurs de mouvement/positionnement et le coussinet comprendrait trois capteurs de pression. Cette technologie aurait pour avantage de chercher à quantifier le déplacement du bras ainsi que la pression qui est exercée localement sur le coussinet. Cependant, il ne semble pas que ces mesures pourraient permettre d'effectuer une analyse simple et quantitative de la flexibilité de la colonne vertébrale d'un point de vue mécanique, puisque ce système ne donne pas accès aux efforts de réaction produit sur chaque bras. De plus, la reproductibilité dans le temps des mesures sur un même sujet semble difficile à assurer. Enfin, les capteurs de pression se trouvent dans le champ de rayons X lors de la prise de radiographie par rayons X, ce qui implique une surdose significative de rayons X par rapport à la même prise de radiographie sans capteur de pression dans le coussinet.

Ainsi aucune des méthodes radiographiques préopératoires actuelles pour évaluer la réductibilité des courbures SIA n'apporte une entière satisfaction aux praticiens.

### 3. Objectifs de l'invention

Un objectif de la présente invention est de pallier au moins certains inconvénients des méthodes actuelles, qu'il s'agisse du segment articulaire du rachis, tel que détaillé ci-dessus, ou bien qu'il s'agisse d'autres segments articulaires, telles les articulations du poignet, du coude, du genou, de la hanche, de l'épaule ou encore de la cheville.

Un objectif de l'invention est de fournir un dispositif médical pour contraindre un segment articulaire humain de façon quantifiée et dans la limite de tolérance du patient. Plus précisément, un objectif de l'invention est notamment de fournir un dispositif médical permettant de contraindre de manière précise, fiable et reproductible un segment articulaire humain.

Un autre objectif de l'invention est de fournir un dispositif médical qui puisse être utilisé pour la radiographie par rayons X, notamment qui permette la prise de radiographie sans irradiation excessive pour le patient.

Un autre objectif de l'invention est, au moins selon certains modes de réalisation, de fournir un dispositif médical simple, relativement peu coûteux et facilement adaptable avec du matériel médical actuellement disponible.

Un autre objectif de l'invention est de proposer un système comprenant le dispositif médical.

Un autre objectif de l'invention est de proposer un procédé pour contraindre un segment articulaire humain.

Un autre objectif de l'invention est de proposer des utilisations spécifiques du dispositif/système médical.

### 4. Exposé de l'invention

La présente invention concerne un dispositif médical pour contraindre un segment articulaire d'un corps humain, comprenant au moins trois unités d'appui destinées à être fixées sur un support.

Chaque unité d'appui comprend :
- un bras extensible ;
- un moyen de mesure d'une force fixé audit bras extensible, adapté pour mesurer un effort s'exerçant sur le bras extensible ;
- un moyen de mesure d'un déplacement, adapté pour mesurer le déplacement du bras extensible ;
- un élément d'appui fixé en partie distale du bras extensible, adapté pour être en contact avec une partie du corps humain et permettant d'appliquer à la partie du corps humain l'effort mesuré par le moyen de mesure d'une force ; et,
- un moyen de fixation, adapté pour la fixation de l'unité d'appui sur le support.

On entend par « segment articulaire », une articulation ou un ensemble d'articulations adjacentes les unes aux autres sur un segment corporel.

Ainsi, les au moins trois unités d'appui du dispositif selon l'invention peuvent être positionnées sur un support et sont destinées à exercer un effort sur plusieurs parties d'un corps humain, permettant ainsi d'en contraindre un segment articulaire. Le bras extensible de chaque unité d'appui permet d'exercer une force d'appui sur une partie du corps humain lorsque l'unité d'appui est en contact au niveau de l'élément d'appui avec cette partie du corps humain. Le moyen de mesure d'une force et le moyen de mesure d'un déplacement permettent de mesurer quantitativement l'effort exercé sur cette partie du corps humain en fonction de l'amplitude de déplacement du bras extensible. Le moyen de mesure d'une force fixé au bras extensible selon l'invention présente comme avantage par rapport aux capteurs de pression utilisés dans l'art antérieur de rendre possible, fiable et reproductible la mesure de l'effort en s'affranchissant totalement de la surface de contact du corps humain avec l'élément d'appui. Le dispositif selon l'invention permet donc de quantifier, de manière précise, fiable et reproductible, les déplacements et les efforts (résultantes et moments) s'exerçant sur un segment articulaire soumis à une contrainte chez un sujet pouvant être dans différentes positions, notamment couché, debout ou encore assis.

Au moins trois unités d'appui composent le dispositif selon l'invention. En effet, trois unités d'appuis destinées à être fixées sur le support permettent de réaliser des contraintes simples en flexion de segments articulaires humains. Quatre unités d'appui peuvent être avantageusement utilisées pour réaliser des contraintes multiples en flexion sur un segment articulaire humain. L'utilisation de quatre unités d'appui est particulièrement appropriée pour exercer des contraintes complexes, notamment sur des rachis fortement déformés, en particulier des rachis diagnostiqués avec scoliose présentant une courbure et une contre-courbure.

Chaque unité d'appui du dispositif selon l'invention comprend un bras extensible. Le bras extensible permet d'exercer une force sur une partie du corps humain lorsque l'unité d'appui est en contact au niveau de l'élément d'appui avec cette partie du corps humain. Par exemple, dans le cas d'un segment articulaire étant le rachis, le bras extensible permet d'exercer une force sur une partie du tronc, notamment sur une partie supérieure, médiane, ou inférieure du tronc, dans un plan frontal ou dans un plan sagittal.

Le bras extensible comprend tout moyen permettant d'exercer une poussée. Avantageusement, le bras extensible peut générer une force d'appui comprise entre 0 et 1000 Newtons.

Préférentiellement, le bras extensible comprend une tige destinée à être guidée longitudinalement en translation selon une direction rectiligne. Le bras extensible peut alors s'étendre selon une direction rectiligne. Le fait que la force soit exercée selon l'axe de la tige est particulièrement avantageux dans la mesure où la force exercée sur la partie du corps humain, après un positionnement précis de la direction de la tige, est alors une force purement de compression de la tige. Le vecteur force exercé sur la partie du corps humain en contact avec l'élément d'appui est alors parfaitement caractérisé puisque sa direction, son sens et sa norme sont parfaitement définis. Le fait de faire travailler le bras extensible en compression pure permet de mieux maîtriser, à tout le moins de maîtriser plus facilement, la bonne reproductibilité des mesures de déplacement et d'effort pour un sujet donné ainsi que l'analyse des propriétés mécaniques, notamment la flexibilité, de sa colonne vertébrale.

Selon un premier mode de réalisation, la tige est guidée par une liaison de type glissière ou de type pivot-glissant. Dans ce mode de réalisation, le bras extensible peut notamment être une tige à poussée manuelle guidée par une liaison de type glissière ou par une liaison de type pivot-glissant, un vérin pneumatique, un vérin électrique ou un vérin hydraulique.

Selon un deuxième mode de réalisation, la tige est guidée par une liaison de type hélicoïdale. Dans ce mode de réalisation, le bras extensible peut notamment être un système vis-écrou ou un système de vis à billes. La mise en mouvement du système vis-écrou peut se faire manuellement ou être motorisée.

Chaque unité d'appui du dispositif selon l'invention comprend un moyen de mesure d'une force. Pour une unité d'appui donnée, ce moyen de mesure d'une force est fixé au bras d'appui et permet de mesurer l'effort s'exerçant sur le bras extensible. Comme déjà mentionné, le moyen de mesure d'une force selon l'invention présente comme avantage par rapport aux capteurs de pression utilisés dans l'art antérieur de rendre la mesure de l'effort de réaction possible de façon fiable et reproductible en s'affranchissant totalement de la surface de contact du corps humain avec l'élément d'appui.

La résolution du moyen de mesure d'une force est préférentiellement au minimum de l'ordre du Newton.

Le moyen de mesure d'une force peut être notamment choisi parmi: les capteurs de force à jauges de déformation, les capteurs de force piézoélectriques, les capteurs de force « type S », les couplemètres, les capteurs de forces en montage sur flasque et les capteurs de force cylindriques creux.

Avantageusement, le moyen de mesure d'une force est un capteur de force de compression cylindrique creux. Le capteur de force de compression cylindrique creux est fixé à une partie destinée à être immobile du bras extensible ainsi qu'au reste de la structure de l'unité d'appui, destinée également à être immobile. L'utilisation d'un capteur de force de compression cylindrique creux a notamment pour avantage de permettre d'éloigner le moyen de mesure de force de la zone où s'exerce l'appui et de diminuer l'encombrement de l'unité d'appui tout en assurant la mesure de l'effort de compression pure appliqué dans la tige. Le capteur de force de compression cylindrique creux peut notamment être positionné de manière suffisamment éloignée de la zone où s'exerce l'appui afin de ne pas interférer avec l'instrumentation de radiographie par rayons X. En effet, la présence dans le champ de la radiographie par rayons X du moyen de mesure d'une force, fabriqué essentiellement en matériau métallique, modifierait sensiblement le calibrage de l'instrumentation de radiographie par rayons X (augmentation de la puissance du faisceau de rayons X lors de la radiographie) et impliquerait une surexposition non-nécessaire du patient aux rayons X.

Chaque unité d'appui du dispositif selon l'invention comprend un moyen de mesure d'un déplacement. Le moyen de mesure d'un déplacement est adapté pour mesurer le déplacement du bras extensible. Le moyen de mesure d'un déplacement peut notamment être choisi parmi : une échelle graduée, un cadran gradué, un capteur laser de déplacement, un palpeur, un comparateur, un capteur sans contact. Préférentiellement, la résolution du moyen de mesure de déplacement est de l'ordre du millimètre.

Avantageusement, le moyen de mesure de déplacement est positionné de manière suffisamment éloignée de la zone où s'exerce l'appui afin de ne pas interférer avec l'instrumentation de radiographie par rayons X. Selon un mode particulier de réalisation de l'invention, le bras extensible est un système vis-écrou, la vis étant destinée à s'étendre selon une direction rectiligne, l'écrou restant immobile par rapport au support. Dans ce mode de réalisation, le moyen de mesure d'un déplacement est avantageusement un compteur du nombre de tours de la vis, le déplacement obtenu pour un tour complet de vis correspondant au pas de la vis.

Chaque unité d'appui du dispositif selon l'invention comprend aussi un élément d'appui fixé en partie distale du bras extensible. L'élément d'appui est adapté pour et, destiné à, être en contact avec une partie du corps humain. L'élément d'appui peut avantageusement être fabriqué dans un polymère souple. De plus, l'élément d'appui peut notamment avoir une forme adaptée au segment corporel du corps humain sur lequel il est destiné à former un appui. Une forme adaptée est notamment une forme permettant une bonne répartition des forces sur une surface suffisamment importante d'un segment corporel humain. L'élément d'appui peut avoir des formes très variées telles, une forme plane, une forme cylindrique, une forme sphérique, une forme convexe ou encore une forme concave.

Finalement, chaque unité d'appui du dispositif selon l'invention comprend un moyen de fixation adapté pour la fixation de l'unité d'appui sur un support. La nature du moyen de fixation dépend de la nature du support. Dans un mode de réalisation particulier, le moyen de fixation est amovible et peut notamment être choisi parmi : un étau, un cavalier, une bride, un collier, une pince, un serre-joint et une sauterelle. Avantageusement, le moyen de fixation comprend une sauterelle. Ceci présente notamment comme avantage que les unités d'appui du dispositif selon l'invention peuvent être fixées sur, ou retirées du, support de manière simple et rapide.

Selon un mode avantageux de l'invention, chaque unité d'appui comprend en outre une liaison pivot ou une liaison pivot glissant selon un axe. La liaison pivot ou la liaison pivot glissant permet d'ajuster la position du bras extensible et/ou de l'élément d'appui angulairement et en hauteur par rapport audit axe. L'élément de blocage permet de bloquer réversiblement la position angulaire et en hauteur du bras extensible et/ou de l'élément d'appui. Avantageusement, chaque unité d'appui comprend également un moyen de mesure angulaire de la position du bras extensible et/ou de l'élément d'appui par rapport audit axe. Ceci a pour avantage de permettre de mesurer précisément la direction de la force d'appui. Le moyen de mesure angulaire peut notamment être choisi parmi : un cadran gradué ou un goniomètre numérique. Préférentiellement, la résolution du moyen de mesure angulaire est de l'ordre du degré.

Selon une caractéristique préférentielle de l'invention, des éléments du dispositif destinés à être dans le champ de rayons X d'une radiographie par rayons X sont fabriqués en matériaux radio-transparents. Les matériaux radio-transparents n'apparaissent pas ou peu sur les clichés de radiographie par rayons X. Les matériaux radio-transparents sont notamment des polymères choisis parmi les polyamides, les polytétrafluoroéthylènes (PTFE), les poly(méthacrylates de méthyle) (PMMA), les polyuréthanes (PU), les polycarbonates (PC), les polychlorures de vinyle (PVC), les polyéthylènes (PE), les mousses de polymère(s) et les polymères élastomères. Les éléments du dispositif destinés à être dans le champ de rayons X d'une radiographie par rayons X sont notamment le bras extensible, en particulier son extrémité distale, et l'élément d'appui de chaque unité d'appui. Ceci permet en particulier que la prise de radiographie par rayons X avec des unités d'appui soit effectuée sans impliquer de surdose significative de rayons X par rapport à une même prise de radiographie sans les unités d'appui, tout en permettant d'obtenir un cliché radiographique de bonne qualité.

L'invention concerne également un système pour contraindre un segment articulaire d'un corps humain. Le système comprend un dispositif médical tel que décrit ci-dessus et un support. Les unités d'appui sont fixées, de manière amovible ou non, sur le support. Au moins une des unités d'appui est destinée à former un appui contre une partie du corps humain. Au moins une autre des unités d'appui est destinée à former un contre-appui contre une autre partie du corps humain.

On entend par « contre-appui » une force ayant un sens sensiblement opposé à un « appui ».

Le support est adapté en fonction de la nature du segment articulaire humain et du patient.

Le support peut notamment être une table de radiologie, une table d'examen, une table d'opération, en position horizontale, inclinée, ou verticale ou encore un portique, un fauteuil roulant, dans le cas d'un segment articulaire étant le rachis.

Le support peut notamment être un fauteuil roulant, un ergomètre, une tablette adaptée ou non pour la radiologie, dans le cas d'un segment articulaire choisi parmi les articulations du poignet, les articulations du coude, les articulations de l'épaule, les articulations des hanches, les articulations du genou et les articulation des chevilles.

Selon un mode particulier de réalisation de l'invention, le support est un support sensiblement plan. On entend par « support sensiblement plan », un support dont une surface est visiblement plane, malgré les aspérités et autres défauts que peut présenter ladite surface. Selon ce mode de réalisation de l'invention, le système pour contraindre un segment articulaire du corps humain comprend : un dispositif médical pour contraindre le segment articulaire humain comprenant au moins trois unités d'appui et un support sensiblement plan. Les unités d'appui sont fixées sur les bords latéraux du support sensiblement plan. Chaque unité d'appui comprend préférentiellement :
- un bras extensible adapté pour s'étendre selon une direction rectiligne dans un plan sensiblement parallèle à celui du support sensiblement plan ;
- un moyen de mesure d'une force fixé audit bras extensible, adapté pour mesurer l'effort s'exerçant sur le bras extensible selon ladite direction rectiligne ;
- un moyen de mesure d'un déplacement, adapté pour mesurer le déplacement rectiligne du bras extensible;
- un élément d'appui fixé en partie distale du dit bras extensible, adapté pour être en contact avec une partie d'un corps humain et permettant d'appliquer à la partie du corps humain l'effort mesuré par le moyen de mesure ; et,
- un moyen de fixation, permettant de fixer l'unité d'appui sur les bords latéraux du support;

Selon ce mode de réalisation, chaque unité d'appui du dispositif comprend avantageusement en outre : une liaison pivot ou une liaison pivot glissant selon un axe sensiblement orthogonal au plan du support, permettant d'ajuster la position du bras extensible et/ou de l'élément d'appui angulairement et/ou en hauteur par rapport audit axe, un élément de blocage permettant en outre de bloquer réversiblement la position angulaire et/ou en hauteur du bras extensible et/ou de l'élément d'appui.

Selon ce mode de réalisation, le moyen de fixation de chaque unité d'appui est préférentiellement amovible, de sorte que chaque unité d'appui puisse être ajustée en position sur les bords latéraux du support en fonction de la morphologie du corps humain.

L'invention concerne également un procédé pour contraindre un segment articulaire d'un corps humain. Le procédé comprend une étape de positionnement des unités d'appui d'un système selon la présente invention, tel que précédemment décrit. L'étape de positionnement des unités d'appui est effectuée de sorte à mettre en contact les éléments d'appui desdites unités d'appui avec des parties du corps humain. Le procédé comprend

Avantageusement, l'étape d'exercice de contrainte(s) est en outre contrôlée par mesure de la position angulaire du bras extensible.

L'invention concerne également l'utilisation d'un système tel que ci-dessus décrit pour étudier la laxité ligamentaire, ou flexibilité, d'un segment articulaire humain. Le segment articulaire humain peut notamment être choisi parmi les articulations du poignet, les articulations du coude, les articulations de l'épaule, les articulations du rachis, les articulations des hanches, les articulations du genou et les articulations de la cheville. Du fait de la bonne maîtrise de la précision et de la reproductibilité des mesures de déplacement et d'effort du bras extensible des unités d'appui, ainsi que du bon contrôle de la direction des forces exercées par les unités d'appui, une analyse précise et reproductible de la flexibilité et du comportement mécanique du segment articulaire est possible.

La présente description décrit en outre l'utilisation d'un système tel que décrit ci-dessus pour prédire de façon pré-opératoire la correction pouvant être attendue par opération chirurgicale pour un rachis humain présentant une déformation.

### 6. Liste des figures

L'invention, ainsi que les différents avantages qu'elle présente, seront plus facilement compris grâce à la description qui va suivre d'un mode non limitatif de réalisation de celle-ci, donnée en référence aux dessins, dans lesquels :
Les Figure 1A et Figure 1B représentent une unité d'appui d'un dispositif, selon la présente invention, fixée à un support et destinée notamment à former appui contre une partie du tronc; la Figure 1A est une vue en perspective de l'unité d'appui alors que la Figure 1B est une coupe de profil de la même unité d'appui.
La Figure 2 représente une vue de dessus d'un système comprenant i) un dispositif comprenant trois unités d'appui telles que représentées en Figure 1A et ii) une table de radiologie.
Les Figures 3A et 3 B représentent un système comprenant i) un dispositif comprenant quatre unités d'appui telles que représentées en Figure 1A et ii) un support étant une table de radiologie (vue de dessus, Figure 3A) ou un portique (vue de face, Figure 3B).
La Figure 4 représente un positionnement approprié de quatre unités d'appui sur un support dans le cas d'un rachis présentant deux courbures.

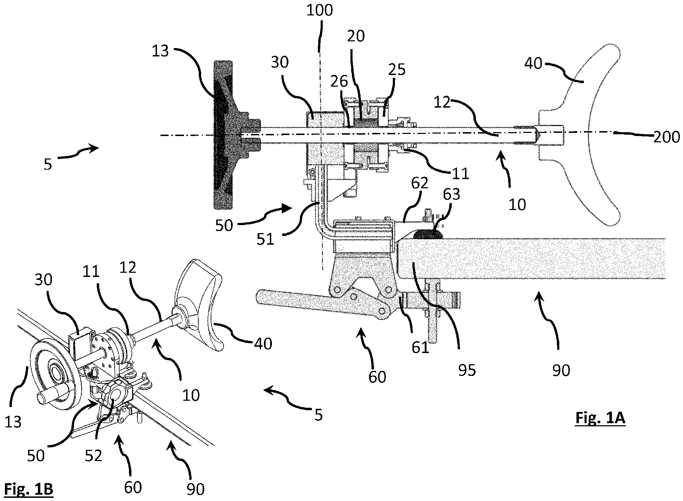

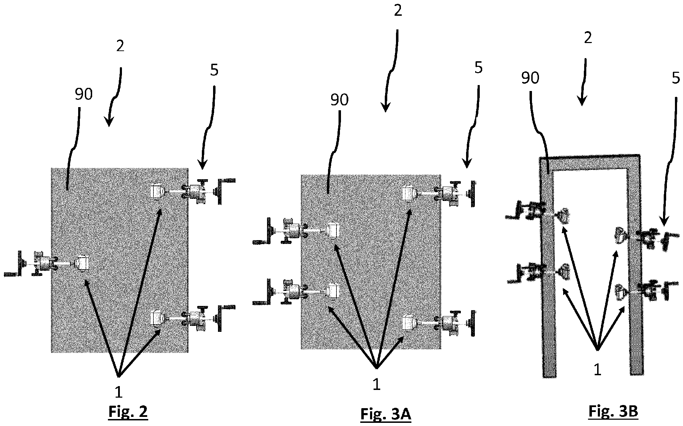

Les Figures 3A et 3 B représentent un système comprenant i) un dispositif comprenant quatre unités d'appui telles que représentées en Figure 1A et ii) un support étant une table de radiologie (vue de dessus, Figure 3A) ou un portique (vue de face, Figure 3B).

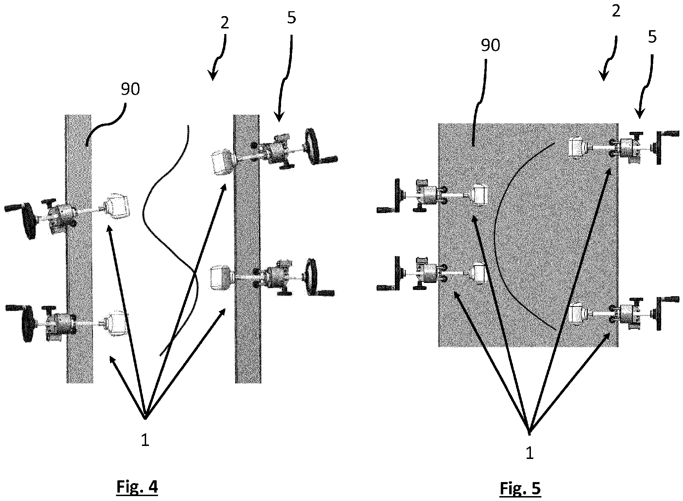

La Figure 4 représente un positionnement approprié de quatre unités d'appui sur un support dans le cas d'un rachis présentant deux courbures.
La Figure 5 représente un positionnement approprié de quatre unités d'appui sur un support dans le cas d'un rachis présentant une seule courbure.

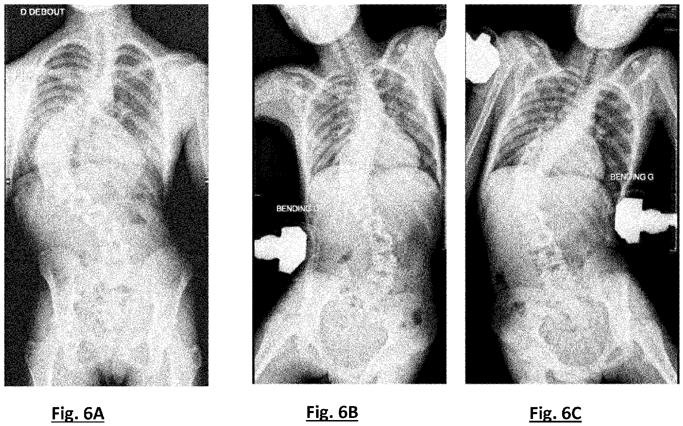

Les Figures 6A, 6B et 6C représentent des clichés de radiographie par rayonx X du tronc d'un patient dans le plan frontal, sans contrainte (Figure 6A), avec flexion latérale droite (Figure 6B, « *Bending D* ») et avec flexion latérale gauche (Figure 6C, « Bending G »).

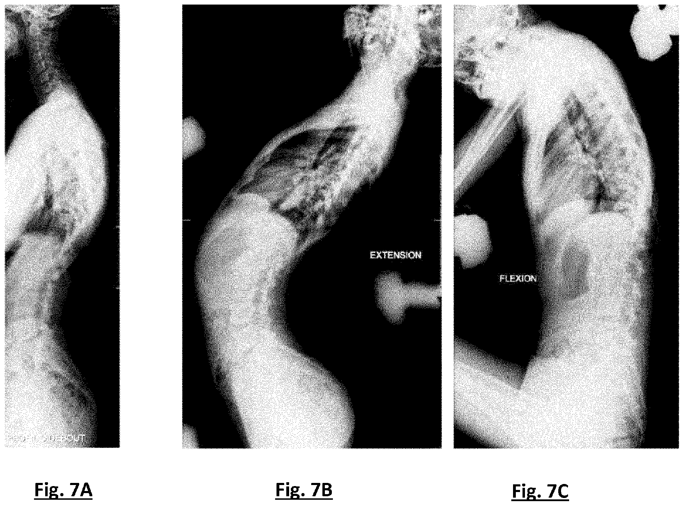

Les Figures 7A, 7B et 7C représentent des clichés de radiographie par rayons X du tronc d'un patient dans le plan sagittal, sans contrainte (Figure 7A), avec flexion avant (Figure 7B) et avec extension arrière (Figure 7C) ;

### 6. Description détaillée d'un mode de réalisation

### 6. 1 Exemple d'unité d'appui adaptée pour la contrainte du rachis

En référence à la Figure 1A et à la Figure 1B, une unité d'appui 5 peut être fixée de manière amovible sur les bords latéraux 95 d'une table de radiologie 90. Chaque unité d'appui 5 comprend : un bras extensible 10, un capteur de force de compression cylindrique creux 20, un capteur laser de déplacement 30, un élément d'appui 40 et un moyen de fixation 60.

Le bras extensible 10 comprend un palier 25 d'acier, un écrou 11, une vis 12 ayant un axe 200 et une manivelle 13. L'écrou 11 est normalement solidarisé au palier 25. La manivelle 13, est fixée à l'extrémité proximale de la vis 12, et permet de faire tourner la vis 12 dans l'écrou 11 : la vis 12 peut ainsi être déplacée selon une direction rectiligne, l'écrou restant immobile par rapport au reste de l'unité d'appui 5. La vis 12 utilisée est de type M16 et a un pas de vis de 2mm. Le système vis 12 - écrou 11 du bras extensible 10 présente de nombreux avantages : le praticien peut le manipuler aisément et peut se faire une idée assez précise du déplacement en comptant le nombre de tours de manivelle 13 donnés, sans même avoir besoin de consulter le capteur laser de déplacement 30. De plus, pour une position donnée de la vis 12 dans laquelle une force longitudinale est appliquée à la vis 12, la position de cette dernière reste inchangée quelle que soit la force exercée : ceci assure en particulier une bonne précision et une bonne fiabilité du déplacement imposé par le praticien au bras extensible 10 ainsi qu'une bonne répétabilité de l'effort exercé par le bras extensible 10 sur un même patient pour une partie du corps donnée.

L'écrou 11 peut temporairement être désolidarisé du palier 25 afin de laisser le praticien pouvoir positionner grossièrement la vis 12 dans une position souhaitée par simple poussée sans devoir effectuer un nombre trop important de tours de manivelle 13. Le palier 25 possède de plus un revêtement en téflon 26 au niveau des points pouvant être en contact avec la vis 12 : ceci sert à garantir une bonne liaison pivot glissant entre la vis 12 et le palier 25 lorsque l'écrou 11 est temporairement désolidarisé du palier 25.

Le capteur de force de compression cylindrique creux 20 est solidarisé au palier 25 et permet de mesurer l'effort s'exerçant sur la vis 12 selon l'axe 200. Il a une résolution au minimum de l'ordre du Newton.

Le capteur laser de déplacement 30 est également solidarisé au palier 25 et permet de mesurer l'amplitude du déplacement rectiligne de la vis 12 selon l'axe 200.

L'élément d'appui 40 est fixé en partie distale de la vis 12. Il a une forme concave adaptée pour être en contact avec des parties du tronc d'un corps humain, notamment des parties supérieure, médiane ou inférieure du tronc, dans le plan frontal ou dans le plan sagittal. La forme concave permet une bonne répartition de la contrainte exercée par le bras extensible 10 sur une partie quelconque du tronc. Evidemment, pour la contrainte d'autres segments articulaires, l'élément d'appui 40 pourra avoir une forme différente et un dimensionnement différent en fonction de la partie du corps sur laquelle il est envisagé d'exercer une contrainte.

Le moyen de fixation 60 est un moyen de fixation amovible. Il comprend : une sauterelle 61, de type horizontal, fixée à un profil latéral 62 muni d'un pied 63. Le moyen de fixation 60 a pour avantage de permettre de fixer ou retirer facilement et rapidement l'unité d'appui 5 de la table de radiologie 90. Une règle graduée (non représentée), peut également être disposée le long des bords latéraux 95 de la table de radiologie 90 afin de connaître la position transversale de l'unité d'appui 5 sur les bords latéraux 95 de la table de radiologie 90. Une alternative (non représentée) de moyen de fixation 60 serait un rail de coulissement fixé sur les bords latéraux 95 de la table de radiologie 90, permettant l'ajustement de la position transversale de l'unité d'appui 5 et, un moyen de blocage, par exemple de type vis, permettant de bloquer l'unité d'appui en une position transversale donnée.

Une liaison pivot glissant 50, entre le moyen de fixation 60 et le palier 25 permet d'ajuster la hauteur et la position angulaire du bras extensible 10 par rapport à une normale 100 à la table de radiologie 90. La liaison pivot glissant 50 est formée par un centrage long sur un tube cintré 51. Le blocage du bras extensible 10 à une hauteur donnée et dans une position angulaire donnée se fait par serrage d'une vis sur le tube cintré par le biais d'un volant à six lobes 52. La hauteur et la position angulaire du bras extensible 10 peuvent être mesurés par des cadrans gradués (non représentés).

Etant donné que le système d'acquisition de radiographie par rayons X calibre sa puissance de rayonnement automatiquement en fonction de la densité de matériaux à traverser, le palier 25, l'écrou 11, le capteur de force de compression cylindrique creux 20 et le capteur laser de déplacement 30 sont disposés de telle sorte que lorsque l'unité d'appui 5 est fixée sur les bords latéraux 95 d'une table de radiologie 90, ils se trouvent également vers les bords latéraux 95 de la table de radiologie 90, de préférence vers l'extérieur. Le palier 25, l'écrou 11, le capteur de force de compression cylindrique creux 20 et le capteur laser de déplacement 30 sont alors hors du champ des rayons X lors de la prise d'une radiographie par rayons X. De plus, les éléments étant destinés à être dans le champ de rayons X lors de la prise d'une radiographie par rayons X, en particulier la vis 12 et l'élément d'appui 40, sont fabriqués en matériaux radio-transparents. La vis 12 est fabriquée en polyamide. L'élément d'appui 40 est fabriqué en polyméthacrylate de méthyle. Ainsi les éléments qui absorberaient beaucoup les rayons X sont déportés en dehors du champ des rayons X, des éléments absorbant peu de rayons X étant utilisés dans le champ des rayons X. Ceci permet en particulier que la prise de radiographie par rayons X avec des unités d'appui 5 fixées sur la table de radiologie 90 soit effectuée sans impliquer de surdose significative de rayons X par rapport à une même prise de radiographie sans les unités d'appui 5, tout en permettant d'obtenir un cliché radiographique de bonne qualité.
Ainsi, le dispositif comprenant trois ou quatre unités d'appui 5 permet de contraindre un rachis dans une forme donnée et de disposer d'informations quantitatives personnalisées des efforts et déplacements mesurés pendant la prise de radiographie par rayons X. Les unités d'appui 5 sont fabriquées avec des éléments simples, adaptables à tout type de support, notamment des tables de radiologie. Il peut en outre être envisagé qu'un patient en fauteuil roulant puisse effectuer une radiographie par rayons X en *stress* assis dans son fauteuil roulant.

### 6.2 Exemple de système adapté pour la contrainte du rachis.

En référence aux Figures 2 et 3A, un système 2 comprend : un dispositif 1 comprenant trois (cf. Figure 2) ou quatre (cf. Figure 3A) unités d'appui, telles que décrites en section 6.1, fixées à une table de radiologie 90.

Dans le cas de trois unités d'appui 5 (cf. Figure 2), l'unité d'appui 5 au centre est disposée pour former un appui et les deux unités d'appui 5 aux extrémités sont disposées pour former un contre-appui (ou inversement).

L'utilisation de quatre unités d'appuis par rapport à trois unités d'appui présente plusieurs avantages. La contrainte imposée est plus homogène entre les appuis : il y a moins de variations de contraintes engendrées dans le segment articulaire et donc moins de singularité de chargement pour le patient, entrainant plus de facilité à corriger et contraindre le segment articulaire (meilleure acceptabilité et tolérance au chargement pour le patient) ; il y a de plus une interchangeabilité facilitée de la forme et de la taille des quatre points d'appui sans répercussion sur la qualité de la sollicitation et de la mesure. Les positionnements des quatre appuis sont modifiables indépendamment et ajustables selon la morphologie du patient et de la déformation du segment articulaire souhaitée.

En particulier, dans le cas de la contrainte du rachis, les quatre unités d'appui 5 peuvent être disposées différemment selon le type de déformation.

En référence à la Figure 4, dans le cas d'une déformation de la colonne à deux courbures (une courbure et une contre-courbure), les quatre unités d'appui 5 peuvent être positionnées comme suit afin d'obtenir un redressement de la colonne vertébrale :
- une unité d'appui 5 sur chacun des deux sommets de courbure ;
- une unité d'appui 5 à l'extrémité proximale de la déformation globale ; et,
- une unité d'appui 5 à l'extrémité distale de la déformation globale.

Un avantage de l'utilisation de quatre unités d'appui par rapport à l'utilisation de trois unités d'appui est que, dans le cas des quatre unités d'appui, la courbure et la contre-courbure peuvent être contraintes simultanément (un seul cliché radiographique) alors que dans le cas de trois unités d'appui, chaque courbure doit être contrainte séparément (deux clichés radiographiques). De plus, la contrainte simultanée de la courbure et la contre courbure permet de prendre en compte les effet de la correction de la courbure sur la contre-courbure et inversement.

En référence à la Figure 5, dans le cas d'une déformation de la colonne à une seule courbure, les quatre unités d'appui peuvent être positionnées comme suit afin d'obtenir un redressement de la colonne vertébrale :
- deux unités d'appui 5 placées de part et d'autre du sommet de la courbure ;
- une unité d'appui 5 à l'extrémité proximale de la déformation globale ; et,
- une unité d'appui 5 à l'extrémité distale de la déformation globale.
La configuration représentée à la Figure 5 peut être utilisée pour la contrainte de la plupart des autres articulations que le rachis.

Les unités d'appui 5 sont positionnées en fonction de la nature du segment articulaire, des déformations souhaitées du segment articulaire et de la physiologie du patient sur des parties adéquates du corps du patient.

Comme représenté aux figures 3A et 3B, des supports possibles sur lesquels les quatre unités d'appui peuvent être fixées sont notamment une table de radiologie (cf. Figure 3A) ou un portique (cf. Figure 3B). La nature du support préférentiellement utilisé dépend des utilisations du dispositif médical. Par exemple, comme il est détaillé dans la section 6.3, il peut être avantageux de recourir à une table de radiologie horizontale sur laquelle le patient peut s'allonger dans le cas d'une utilisation pour corriger la forme d'un rachis en pré-opératoire puisque le patient devra être allongé lors de l'opération de correction. A contrario, il peut être avantageux d'utiliser un portique vertical, le patient devant alors rester debout, dans le cas d'une utilisation pour la fabrication d'un corset visant à corriger la forme d'un rachis, le corset étant destiné à être porté notamment en positions assise ou debout.

### 6.3 Utilisations du système pour contraindre un rachis dans une position donnée

Le système représenté en Figure 2 est utilisé pour contraindre dans une position donnée le rachis d'une personne présentant une déformation du rachis, tout en mesurant quantitativement la direction et la valeur des forces s'exerçant sur chacun des bras extensibles des unités d'appui ainsi que la valeur du déplacement de chacun des bras extensibles des unités d'appui.

Une méthode générale pouvant être utilisée pour diverses utilisations du système représenté en section 6.2 comprend des étapes de:
i) positionnement d'un patient sur le plan frontal ou sagittal ;
ii) prise d'une première radiographie du rachis sans contrainte ;
iii) émission d'un diagnostic concernant la déformation du rachis à partir de la première radiographie ;
iii) positionnement en appui ou en contre-appui des unités d'appuis contre des parties du corps du patient en fonction du diagnostic émis après la première radiographie et en fonction de la morphologie du patient ;
iv) exercice d'une contrainte par au moins une unité d'appui, de manière contrôlée en fonction du relèvement de valeurs quantitatives de positionnement (positionnement et déplacement rectiligne du bras extensible et, position angulaire) et de force s'exerçant sur les unités d'appui : le rachis est alors qualifié comme étant « sous contrainte » ; et,
v) prise d'une seconde radiographie du rachis sous contrainte.
Les étapes iii), iv) et v) peuvent éventuellement être répétées de manière itérative à partir du diagnostic émis à partir du dernier cliché radiographique d'un rachis « sous contrainte ».

La méthode couple donc l'utilisation d'un système selon l'invention avec un appareillage de radiographie par rayons X. L'appareillage de radiographie par rayons X permet de suivre l'évolution de la forme du rachis sans contrainte ou sous divers types de contraintes (dont l'état parfaitement défini par le relèvement de valeurs quantitatives de positionnement et de force s'exerçant sur les unités d'appui). L'appareillage usuel de radiographie par rayons X implique la prise de clichés selon un seul plan, notamment soit le plan sagittal, soit le plan frontal. Des exemples de clichés sont présentés sur les Figures 6A, 6B, 6C, 7A, 7B & 7C. La Figure 6A représente un cliché radiographique sans contrainte d'un rachis dans le plan frontal. Les Figures 6B et 6C représentent des clichés radiographiques sous contrainte du rachis, en flexions latérales respectivement droite et gauche. De manière analogue, la Figure 7A représente un cliché radiographique sans contrainte d'un rachis dans le plan sagittal. Les Figures 7B et 7C représentent des clichés radiographiques sous contrainte du rachis, respectivement en flexion avant et en extension arrière.
Un appareillage plus évolué que l'appareillage usuel de radiographie par rayons X peut également être utilisé, comme un appareil d'imagerie EOS, permettant de couvrir par balayage le plan frontal et le plan sagittal et émettant une dose inférieure de rayons X.

Toutes les mesures quantitatives de positionnement et de force relevées par les différents capteurs que comportent les unités d'appui peuvent être acquises via des cartes d'acquisition par ordinateur afin de faciliter le traitement et l'analyse des données. Il est donc possible avec un même outil informatique de corréler les déplacements et les efforts imposés sur chaque unité d'appui à la déformation du rachis entre le premier cliché radiographique (rachis sans contrainte) et le n^{ème}, notamment deuxième, cliché radiographie (rachis sous contrainte). Ainsi, il est possible de corréler l'analyse biomécanique de la flexibilité et de la correction de la déformation du rachis et l'analyse clinique.

De manière générale, la méthode détaillée ci-dessus peut être utilisée pour la prévention d'escarres liées aux appuis dans un système de contention d'un segment articulaire. En effet, les escarres apparaissent suite à des pressions trop importantes exercées sur des tissus, notamment sur la peau. Les unités d'appuis du dispositif médical permettent notamment d'identifier les parties du corps où s'exerceront le plus de contraintes lors du port du système de contention : le système de contention peut alors être adapté afin de réduire au mieux de trop fortes pressions exercées localement.

En particulier, la méthode détaillée ci-dessus peut permettre d'envisager l'optimisation des appuis dans la fabrication de corsets. Pour une telle utilisation, les radiographies sont avantageusement réalisées en position debout afin d'être au plus proche des conditions d'utilisation du corset. Les unités d'appuis du dispositif médical permettent notamment de déterminer le maximum de correction envisageable ainsi que les parties du corps où s'exerceront le plus de contraintes lors du port du corset, afin de concevoir un corset induisant des conditions physiologiquement acceptables pour le patient et minimisant le risque d'escarres.

La méthode détaillée ci-dessus peut également être utilisée pour prédire de manière pré-opératoire la correction pouvant être attendue par opération chirurgicale du rachis. Le recours à la chirurgie est souvent nécessaire lorsque de fortes déformations du rachis (courbures) ont été diagnostiquées. Cette méthode a pour but d'aider le chirurgien en offrant la possibilité de redresser la colonne vertébrale de façon temporaire permettant la prise de clichés radiographiques dans une ou plusieurs situations de correction afin de prédire la géométrie qu'aura le rachis scoliotique après l'intervention. Cette méthode permet donc de limiter les risques opératoires / post-opératoires induits par de fortes corrections. Elle permet également d'aider le chirurgien dans le choix du matériel de stabilisation et des positionnements des implants sur chaque vertèbre. Elle permet une aide à la décision de l'abord chirurgical : antérieur, postérieur, latéral ou combiné. Elle permet aussi de décider du moment le plus propice à l'intervention.

Cette méthode représente une avancée majeure dans la prédiction des corrections préopératoires comparativement aux méthodes de l'art antérieur. Premièrement, il n'existe pas à la connaissance des inventeurs de tel dispositif/système permettant de réaliser une déformation du rachis *in situ* dans un appareillage de radiographie par rayons X. Deuxièmement, l'ensemble des prédictions peut être réalisée en position physiologique, c'est à dire en position analogue à la position opératoire. Troisièmement, comme le patient n'intervient pas de manière active dans cette méthode, les prédictions de correction sont plus fiables, plus précises et plus reproductibles que celles obtenues dans les méthodes de l'art antérieur.

Ainsi la méthode peut être appliquée sur un patient donné, à qui il a été diagnostiqué une déformation du rachis ou une scoliose (première radiographie, sans contrainte). Une ou plusieurs déformations sont appliquées au rachis du patient, resté vigile, à l'aide du dispositif selon l'invention afin de rechercher une correction satisfaisante (deuxième ou n^{ème} radiographie, sous contrainte) : le dispositif permet d'obtenir des données quantifiées de cette correction, et d'orienter et d'optimiser :
- le choix des dispositifs médicaux implantables ;
- le choix des matériaux à utiliser pour les tiges pour optimiser la rigidité et la résistance en fonction de la correction envisagée : alliages, titane, chrome-cobalt, etc. ;
- le nombre et le positionnement des implants en fonction du nombre d'étages à corriger ;
- les tailles des implants adaptées en fonction des étages à corriger ; et,
- les matériaux des implants.

Le patient est endormi lors de l'intervention chirurgicale et est replacé dans les mêmes conditions de corrections avec une situation identique pour la correction (positions des bras extensibles, déplacement des bras extensibles, angles, forces appliquées). La même correction est donc appliquée qu'en situation vigile limitant fortement les risques de sur-correction et donc de déficit neurologique lors de l'intervention chirurgicale.

### 6.4. Généralisation

Bien que les exemples des sections 6.1, 6.2 et 6.3 se focalisent presque exclusivement sur le rachis, il est admis une généralisation de l'utilisation du dispositif à d'autres segments articulaires, par adaptation dimensionnelle/fonctionnelle des unités d'appui en fonction du segment articulaire d'intérêt et par utilisation d'un support approprié pour fixer le dispositif.

Par exemple, le dispositif peut être utilisé pour les genoux et permet :
- d'identifier le niveau de contrainte d'une prothèse ;
- de choisir le type de prothèse pouvant être utilisé : prothèse contrainte, semi-contrainte ou à charnière ;
- de quantifier les instabilités ligamentaires en s'assurant du contact de chacun des points quel-que-soit la morphologie du patient et de son degré de coopération et de participation ; et
- de s'affranchir de la contracture musculaire pendant l'examen.

## Revendications

1. Dispositif médical (1) pour contraindre un segment articulaire d'un corps humain comprenant au moins trois unités d'appui (5) destinées à être fixées sur un support (90), chaque unité d'appui (5) comprenant :
- un bras extensible (10);
- un moyen de mesure d'un déplacement (30), adapté pour mesurer le déplacement dudit bras extensible (10);
- un moyen de fixation (60), adapté pour la fixation de ladite unité d'appui (5) sur ledit support (90) ;
ledit dispositif médical étant **caractérisé en ce que** chaque unité d'appui (5) comprend en outre :
- un moyen de mesure d'une force (20) fixé audit bras extensible (10), adapté pour mesurer un effort s'exerçant sur ledit bras extensible (10); et,
- un élément d'appui (40) fixé en partie distale dudit bras extensible (10), adapté pour être en contact avec une partie du corps humain et permettant d'appliquer à ladite partie du corps humain ledit effort mesuré par ledit moyen de mesure d'une force (20).

2. Dispositif médical (1) selon la revendication 1, **caractérisé en ce qu'**il comprend quatre unités d'appui (5).

3. Dispositif médical (1) selon l'une quelconque des revendications précédentes **caractérisé en ce que** ledit bras extensible (10) comprend une tige (12) destinée à être guidée longitudinalement en translation selon une direction rectiligne (200).

4. Dispositif médical (1) selon la revendication 3, **caractérisé en ce que** ledit moyen de mesure d'une force (20) est un capteur de force de compression cylindrique creux.

5. Dispositif médical (1) selon l'une quelconque des revendications précédentes, **caractérisé en ce que** chaque unité d'appui (5) comprend une liaison pivot ou une liaison pivot glissant (50) selon un axe (100), permettant d'ajuster la position dudit bras extensible (10) et/ou dudit élément d'appui (40) angulairement et/ou en hauteur par rapport audit axe (100), un élément de blocage (52) permettant en outre de bloquer réversiblement la position angulaire et/ou en hauteur dudit bras extensible (10) et/ou dudit élément d'appui (40).

6. Dispositif médical (1) selon la revendication 5, **caractérisé en ce qu'**il comprend un moyen de mesure angulaire de la position dudit bras extensible (10) et/ou dudit élément d'appui (40) par rapport audit axe (100).

7. Dispositif médical (1) selon l'une quelconque des revendications précédentes, **caractérisé en ce que** des éléments (12, 40) dudit dispositif (1) destinés à être dans le champ de rayons X d'une radiographie par rayons X, notamment l'extrémité distale dudit bras extensible (10) et ledit élément d'appui (40), sont fabriqués en matériaux radio-transparents.

8. Système (2) pour contraindre un segment articulaire d'un corps humain comprenant un dispositif médical (1) selon l'une quelconque des revendications 1 à 7 et un support (90), lesdites unités d'appui (5) dudit dispositif médical (1) étant fixées audit support (90), au moins une desdites unités d'appui (5) étant destinée à former un appui contre une partie dudit corps humain, au moins une autre desdites unités d'appui (5) étant destinée à former un contre-appui contre une autre partie dudit corps humain.

9. Procédé pour contraindre un segment articulaire d'un corps humain, comprenant une étape de positionnement des unités d'appui (5) d'un système (2) selon la revendication 8, de sorte à mettre en contact les éléments d'appui (40) desdites unités d'appui (5) avec des parties dudit corps humain et, une étape d'exercice de contrainte(s) d'au moins une desdites unités d'appui (5) sur au moins une desdites parties du corps humain, ladite étape d'exercice de contrainte(s) étant contrôlée par mesure du déplacement dudit bras extensible (10) et par mesure de l'effort exercé sur ledit bras extensible (10) de chacune desdites unités d'appui (5).

10. Utilisation d'un système (2) selon la revendication 8 pour étudier la laxité ligamentaire d'un segment articulaire d'un corps humain, ledit segment articulaire humain pouvant notamment être choisi parmi les articulations du poignet, les articulations du coude, les articulations de l'épaule, les articulations du rachis, les articulations des hanches, les articulations du genou et les articulations de la cheville.

## Patentansprüche

1. Medizinische Vorrichtung (1) zur Druckausübung auf ein Gelenksegment eines menschlichen Körpers, welche mindestens drei Stützeinheiten (5) umfasst, die dazu bestimmt sind, an einem Träger (90) befestigt zu werden, wobei jede Stützeinheit (5) umfasst:
- einen ausfahrbaren Arm (10);
- ein Messmittel für eine Bewegung (30), das dazu ausgelegt ist, die Bewegung des ausfahrbaren Armes (10) zu messen;
- ein Befestigungsmittel (60), das für die Befestigung der Stützeinheit (5) an dem Träger (90) ausgelegt ist;
wobei die medizinische Vorrichtung **dadurch gekennzeichnet ist, dass** jede Stützeinheit (5) außerdem umfasst:
- ein an dem ausfahrbaren Arm (10) befestigtes Messmittel für eine Kraft (20), das dazu ausgelegt ist, eine Kraft zu messen, die auf den ausfahrbaren Arm (10) ausgeübt wird; und
- ein am distalen Teil des ausfahrbaren Armes (10) befestigtes Stützelement (40), das dazu ausgelegt ist, sich in Kontakt mit einem Teil des menschlichen Körpers zu befinden, und ermöglicht, auf diesen Teil des menschlichen Körpers die Kraft auszuüben, die von dem Messmittel für eine Kraft (20) gemessen wird.

2. Medizinische Vorrichtung (1) nach Anspruch 1, **dadurch gekennzeichnet, dass** sie vier Stützeinheit (5) umfasst.

3. Medizinische Vorrichtung (1) nach einem der vorhergehenden Ansprüche, **dadurch gekennzeichnet, dass** der ausfahrbare Arm (10) eine Stange (12) umfasst, die dazu bestimmt ist, längs in einer geradlinigen Richtung (200) translatorisch geführt zu werden.

4. Medizinische Vorrichtung (1) nach Anspruch 3, **dadurch gekennzeichnet, dass** das Messmittel für eine Kraft (20) ein hohler zylindrischer Druckkraftsensor ist.

5. Medizinische Vorrichtung (1) nach einem der vorhergehenden Ansprüche, **dadurch gekennzeichnet, dass** jede Stützeinheit (5) eine Schwenkverbindung oder eine entlang einer Achse (100) gleitende Schwenkverbindung (50) umfasst, die es ermöglicht, die Position des schwenkbaren Armes (10) und/oder des Stützelements (40) in Winkelrichtung und/oder in der Höhe in Bezug auf die Achse (100) zu verstellen, wobei ein Feststellelement (52) außerdem ermöglicht, die Winkel- und/oder Höhenposition des ausfahrbaren Armes (10) und/oder des Stützelements (40) reversibel zu arretieren.

6. Medizinische Vorrichtung (1) nach Anspruch 5, **dadurch gekennzeichnet, dass** sie ein Messmittel für die Winkelposition des ausfahrbaren Armes (10) und/oder des Stützelements (40) in Bezug auf die Achse (100) umfasst.

7. Medizinische Vorrichtung (1) nach einem der vorhergehenden Ansprüche, **dadurch gekennzeichnet, dass** die Elemente (12, 40) der Vorrichtung (1), die dazu bestimmt sind, sich im Röntgenstrahlenfeld einer Röntgenradiographie zu befinden, insbesondere das distale Ende des ausfahrbaren Armes (10) und das Stützelement (40), aus strahlendurchlässigen Materialien hergestellt sind.

8. System (2) zur Druckausübung auf ein Gelenksegment eines menschlichen Körpers, welches eine medizinische Vorrichtung (1) nach einem der Ansprüche 1 bis 7 und einen Träger (90) umfasst, wobei die Stützeinheiten (5) der medizinischen Vorrichtung (1) an dem Träger (90) befestigt sind, wobei mindestens eine der Stützeinheiten (5) dazu bestimmt ist, eine Abstützung für einen Teil des menschlichen Körpers zu bilden, wobei mindestens eine weitere der Stützeinheiten (5) dazu bestimmt ist, eine Gegenabstützung für einen anderen Teil des menschlichen Körpers zu bilden.

9. Verfahren zur Druckausübung auf ein Gelenksegment eines menschlichen Körpers, welches einen Schritt der Positionierung der Stützeinheiten (5) eines Systems (2) nach Anspruch 8 umfasst, um die Stützelemente (40) der Stützeinheiten (5) mit Teilen des menschlichen Körpers in Kontakt zu bringen, und einen Schritt der Ausübung von Druck (Drücken) mindestens einer der Stützeinheiten (5) auf mindestens eines der Teile des menschlichen Körpers, wobei der Schritt der Ausübung von Druck (Drücken) durch Messung der Bewegung des ausfahrbaren Armes (10) und durch Messung der auf den ausfahrbaren Arm (10) jeder der Stützeinheiten (5) ausgeübten Kraft kontrolliert wird.

10. Verwendung eines Systems (2) nach Anspruch 8 zur Untersuchung der Schlaffheit der Ligamente eines Gelenksegments eines menschlichen Körpers, wobei dieses menschliche Gelenksegment insbesondere aus den Gelenken der Handwurzel, den Gelenken des Ellbogens, den Gelenken der Schulter, den Gelenken der Wirbelsäule, den Gelenken der Hüften, den Gelenken des Knies und den Gelenken des Knöchels ausgewählt sein kann.

## Claims

1. A medical device (1) for applying pressure to a joint segment of a human body comprising at least three bearing units (5) intended to be attached to a holder (90), each bearing unit (5) comprising:
- an extendable arm (10);
- a movement-measuring means (30), adapted to measure the movement of said extendable arm (10);
- an attachment means (60), adapted to attach said bearing unit (5) to said holder (90);
said medical device being **characterised in that** each bearing unit (5) further comprises:
- a force-measuring means (20) attached to said extendable arm (10), adapted to measure a force exerted on said extendable arm (10); and,
- a bearing element (40) attached to the distal portion of said extendable arm (10), adapted to be in contact with a part of the human body and making it possible to apply said force measured by said force-measuring means (20) to said part of the human body.

2. A medical device (1) according to claim 1, **characterised in that** it comprises four bearing units (5).

3. A medical device (1) according to any one of the preceding claims, **characterised in that** said extendable arm (10) comprises a rod (12) intended to be guided longitudinally in translation in a rectilinear direction (200).

4. A medical device (1) according to claim 3, **characterised in that** said force-measuring means (20) is a hollow cylindrical compression force sensor.

5. A medical device (1) according to any one of the preceding claims, **characterised in that** each bearing unit (5) comprises a pivot connection or sliding pivot connection (50) along an axis (100), allowing the position of said extendable arm (10) and/or said bearing element (40) to be adjusted angularly and/or vertically with respect to said axis (100), a locking element (52) also allowing the angular and/or vertical position of said extendable arm (10) and/or said bearing element (40) to be locked reversibly.

6. A medical device (1) according to claim 5, **characterised in that** it comprises an angular measuring means of the position of said extendable arm (10) and/or said bearing element (40) with respect to said axis (100).

7. A medical device (1) according to any one of the preceding claims, **characterised in that** elements (12, 40) of said device (1) intended to be in the X-ray field of an X-ray radiography, in particular the distal end of said extendable arm (10) and said bearing element (40), are made of radiolucent materials.

8. A system (2) for applying pressure to a joint segment of a human body comprising a medical device (1) according to any one of claims 1 to 7 and a holder (90), said bearing units (5) of said medical device (1) being attached to said holder (90), at least one of said bearing units (5) being intended to constitute a pressure against a part of said human body, at least one other of said bearing units (5) being intended to constitute a counter-pressure against another part of said human body.

9. A method for applying pressure to a joint segment of a human body, comprising a step of positioning the bearing units (5) of a system (2) according to claim 8, so as to bring the bearing elements (40) of said bearing units (5) in contact with parts of said human body and, a step of applying pressure of at least one of said bearing units (5) to at least one of said parts of the human body, said pressure application step being controlled by measuring the movement of said extendable arm (10) and by measuring the force exerted on said extendable arm (10) of each of said bearing units (5).

10. Use of a system (2) according to claim 8 for studying the ligament laxity of a joint segment of a human body, said human joint segment being able to be selected in particular from the joints of the wrist, elbow, shoulder, spine, hips, knee and ankle.
